# EUROPEAN PATENT SPECIFICATION

(11) **EP 2 298 236 B1**
(45) Date of publication and mention of the grant of the patent: **06.11.2013**
(21) Application number: 10178467.6
(22) Date of filing: 02.09.2005
(51) Int. Cl.: A61F 2/01

(54) **Vein filter**
Venenfilter
Filtre de veine

(30) Priority: 27.09.2004 US 613362 P; 27.09.2004 US 613574 P
(43) Date of publication of application: 23.03.2011
(62) Divisional of application: 05793070.3
(73) Proprietor: Rex Medical, L.P., Conshohocken, PA 19428 (US)
(72) Inventor: McGuckin Jr., James F, Radnor, PA 19087 (US); Thinnes Jr., John H., Miami, FL 33137 (US)
(74) Representative: Jackson, Derek Charles

(56) References cited:
- EP-A1- 0 270 432
- WO-A-99/25252
- WO-A2-2005/102439
- US-A- 5 669 933

## Description

This application relates to a vascular filter and more particularly to a vein filter for capturing blood clots within the vessel.

Passage of blood clots to the lungs is known as pulmonary embolism. These clots typically originate in the veins of the lower limbs and can migrate through the vascular system to the lungs where they can obstruct blood flow and therefore interfere with oxygenation of the blood. Pulmonary embolisms can also cause shock and even death.

In some instances, blood thinning medication, e.g. anticoagulants such as Heparin, or sodium warfare can be given to the patient. These medications, however, have limited use since they may not be able to be administered to patients after surgery or stroke or given to patients with high risk of internal bleeding. Also, this medication approach is not always effective in preventing recurring blood clots.

Therefore, surgical methods to reduce the likelihood of such pulmonary embolisms by actually blocking the blood clot from reaching the lungs have been developed. One surgical method of treatment involved major surgery where the size of the vessel lumen was restricted by placement of ligatures or clips around the vein, e.g. the inferior vena cava which transports blood from the lower portion of the body to the heart and lungs. This prevented passage of dangerously large blood clots through the vein to the lungs. However, this approach is an invasive surgical procedure, requiring an abdominal incision and general anesthesia and frequently causing vessel thrombosis and lower extremity swelling. Also, there is a lengthy patient recovery time and additional hospital and surgeon expenses associated with this major surgery. In fact, oftentimes, the patients requiring the surgery are unhealthy and the major surgery and general anesthesia poses a risk in and of itself.

To avoid such invasive surgery, less invasive surgical techniques have been developed. These involve the placement of a mechanical barrier in the inferior vena cava. These barriers are in the form of filters and are typically inserted through either the femoral vein in the patient's leg or the right jugular vein in the patient's neck or arm under local anesthesia. The filters are then advanced intravascularly to the inferior vena cava where they are expanded to block migration of the blood clots from the lower portion of the body to the heart and lungs.

These prior filters take various forms. One type of filter is composed of coiled wires such as disclosed in U.S. Patent nos. 5,893,869 and 6,059,825. Another type of filter consists of legs with free ends having anchors for embedding in the vessel wall to hold the filter. These filters are disclosed, for example, in U.S. Patent nos. 4,688,553, 4,781,173, 4,832,055, and 5,059,205, 5,984,947 and 6,007,558. Another type of filter is disclosed in U.S. Patent no. 6,214,025 consisting of wires twisted together to form a cylindrical anchoring portion conforming to the inner vessel wall surface to exert a radial force and a conical filtering portion.

Several factors have to be considered in designing vein filters. One factor is that the filter needs to be securely anchored within the vessel wall, while avoiding traumatic engagement and damage to the wall as well as damage to the neighboring abdominal aorta. Another factor is that the filter must be collapsible to a sufficiently small size to be easily maneuvered and atraumatically advanced intravascularly to the inferior vena cava or other target vessel. Thirdly, the filter should direct the blood clots to the center of the vessel to improve dissolution of the clot within the vessel by the blood flow.

It would be advantageous to provide a vein filter that satisfies the foregoing parameters. Namely, such vein filter would advantageously have sufficient anchoring force to retain the filter within the vessel while providing atraumatic contact with the vessel wall, would have a minimized insertion (collapsed) profile to facilitate delivery through the vascular system to the surgical site, and would enable migration of the captured blood clots to the center of the vessel. Moreover, it would also be advantageous to provide a filter that could simplify insertion through the femoral or the right jugular vein or arm into the inferior vena cava.

Additionally, the need for a vein filter in many patients is temporary. In these instances it would be advantageous to provide a vein filter that satisfies the foregoing factors and in addition could be readily removed from the patient. It would further be advantageous if the filter could be removed minimally invasively, e.g. intravascularly.

In addition, in certain applications, it would be advantageous to provide a filter that satisfies the above criteria plus provides a backup for blood clots that could bypass the filtering region.

US-A-5669933 describes a blood clot filter which is collapsible toward a central longitudinal axis into a collapsed configuration for insertion into a blood vessel and which is radially expandable outwardly from the longitudinal axis to an expanded configuration for contact with the inner wall of the blood vessel at two longitudinally spaced locations. A first plurality of spaced, elongate arms, in the expanded configuration of the filter, curve outwardly away from the longitudinal axis toward the trailing end of the filter to form a first filter basket. These arms prevent movement of the filter in a first longitudinal direction. A second plurality of spaced elongate legs angle outwardly away from the longitudinal axis toward the leading edge of the filter in the expanded configuration thereof. These second legs form a second filter basket opening toward the leading end and prevent longitudinal movement of the filter in a second direction opposite to the first direction.

The present invention overcomes the problems and deficiencies of the prior art. The present invention provides a vessel filter comprising a first region and a second region, the filter movable between a collapsed position for delivery to the vessel and an expanded position for placement within the vessel, the first region having a first set of struts forming a first mounting portion and a filter portion opening in a first direction, the filter portion having a first converging region at a first portion to direct particles toward the center of the filter, the mounting portion being flared in the expanded position to have a transverse dimension increasing in a direction away from the first portion, the second region having a second set of struts forming a second mounting portion which is flared in the expanded configuration and opening in the first direction, wherein a plurality of spaced apart struts extend between the first and second regions, the struts being substantially parallel to a longitudinal axis of the filter and being formed from cutouts in the filter, the struts in the second region extending from the cutouts.

In one embodiment, the filter is formed from a laser cut tube and composed of shape memory material. In one embodiment, the struts of the first set of struts terminate in a vessel engaging hook. In one embodiment, the struts of the second set of struts terminate in a vessel engaging hook. Each of the vessel engaging hooks may lie in a common plane with the respective strut.

In one embodiment, the first region includes a retrieval region including a hook having a cutout exposing an internal annular surface dimensioned to receive a portion of a retrieval sheath.

In one embodiment, at least two adjacent struts of the first set of struts are interconnected by strut portions extending towards one another. The at least to adjacent struts may interconnect at a first joining region and then bifurcate into two interconnecting struts, wherein interconnecting struts of adjacent struts join at a second joining region.

The second region may further include a retrieval region, the retrieval region including a hook having a cutout exposing an internal annular surface, the annular surface dimensioned to receive a portion of a retrieval sheath. The retrieval region may include a radiused region having first and second curved surfaces extending distally inwardly.

Preferred embodiment(s) of the present disclosure are described herein with reference to the drawings wherein:

Figure 1 is a perspective view of a first embodiment of the vein filter of the present invention having two filter portions, the filter shown in the collapsed configuration;

Figure 2 is an enlarged side view of a portion of the vein filter of Figure 1;

Figure 3 is a perspective view of the vein filter of Figure 1 in an expanded configuration;

Figure 4 is a side view of the vein filter of Figure 3 in the expanded configuration;

Figure 5A is a front view of the vein filter of Figure 3 in the expanded configuration;

Figure 5B is an enlarged view of the retrieval hook of Figure 1;

Figure 6 is a perspective view of an alternate embodiment of the vein filter of the present invention shown in the expanded configuration;

Figures 7A and 7B are perspective and side views, respectively, of another alternate embodiment of the vein filter of the present invention shown in the expanded configuration;

Figure 8 is a perspective view of another alternate embodiment of the vein filter of the present invention shown in the expanded configuration;

Figures 9, 10, and 11 illustrate delivery and placement of the vessel filter of Figure 1 in the inferior vena cava wherein Figure 9 illustrates initial insertion of the delivery sheath through the femoral vein, Figure 10 illustrates the delivery sheath being advanced toward the inferior vena cava just below (upstream) the juncture of the renal arteries, and Figure 11 illustrates the delivery sheath fully withdrawn to place the filter in the expanded placement configuration in the inferior vena cava;

Figure 12 is a perspective view of another alternate embodiment of the vein filter of the present invention having a filter portion and two mounting portions, the filter shown in the collapsed configuration;

Figure 13 is an enlarged side view of a portion of the vein filter of Figure 12;

Figure 14 is a perspective view of the vein filter of Figure 12 in an expanded configuration;

Figure 15 is a side view of the vein filter of Figure 14 in the expanded configuration;

Figure 16A is a front view of the vein filter of Figure 14 in the expanded configuration;

Figure 16B is an enlarged view of the retrieval hook of Figure 12;

Figure 17 is a perspective view of another alternate embodiment of the vein filter of the present invention shown in the expanded configuration;

Figure 18 is a perspective view of another alternate embodiment of the vein filter of the present invention shown in the expanded configuration;

Figure 19 is a perspective view of another alternate embodiment of the vein filter of the present invention shown in the expanded configuration; and

Figures 20, 21 and 22 illustrate delivery and placement of the vessel filter of Figure 12 in the inferior vena cava wherein Figure 20 illustrates initial insertion of the delivery sheath through the femoral vein, Figure 21 illustrates the delivery sheath being advanced toward the inferior vena cava just below (upstream) the juncture of the renal arteries, and Figure 22 illustrates the delivery sheath fully withdrawn to place the filter in the expanded placement configuration in the inferior vena cava.

Turning now to the drawings, wherein like reference numerals identify similar or like components throughout the several views, the vein filter of the present invention is described for placement within the inferior vena cava to capture blood clots or other particles which could otherwise pass to the lungs.

The filter is movable from a low profile collapsed configuration to facilitate insertion through the delivery sheath to a larger expanded placement configuration to enable atraumatic engagement with the vessel walls to secure (mount) the filter within the inferior vena cava.

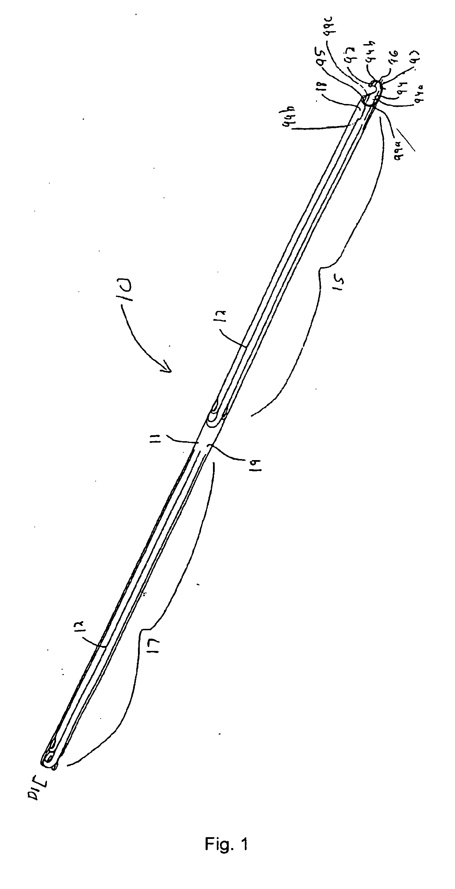

In some embodiments herein, (Figures 1-11) the filter has two substantially bell-shaped regions, each including a filtering region (portion/section) and a flared mounting (anchoring) region (portion/section). As described in more detail below, each filtering portion has inwardly directed struts, terminating in a converging region, thereby directing particles toward the central axis of the filter. By directing the particles to the center and trapping them at the center, they will be exposed to greater blood flow than if trapped at the edges of the filter thereby, improving dissolution of the particles. By providing two filtering portions, particles that bypass the first filtering portion can be captured by the second filtering portion. The flared mounting portion provides less contact area than a straight region, resulting in less tissue ingrowth to facilitate removal of the filter if desired. The flare also reduces the chance of vessel distortion if inserted into a curved vena cava.

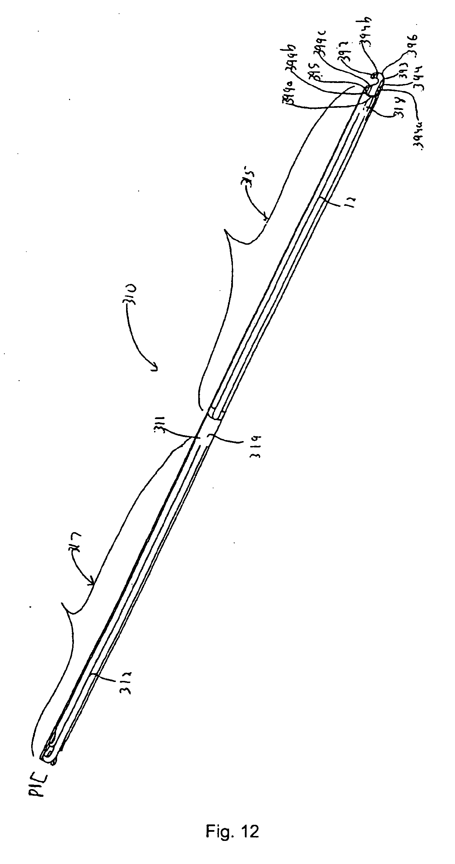

In other embodiments herein (Figures 12-22), the filter has a substantially bell-shaped region having a filtering region (portion/section) and a flared mounting (anchoring) region (portion/section). As described in more detail below, the filtering portion has inwardly directed struts, terminating in a converging region, thereby directing particles toward the central axis of the filter to provide the advantages discussed herein. To enhance mounting of the filter, a second mounting region is provided axially spaced from the bell-shaped region. This second region provides a stabilizing portion to help center the filter and limit tilting.

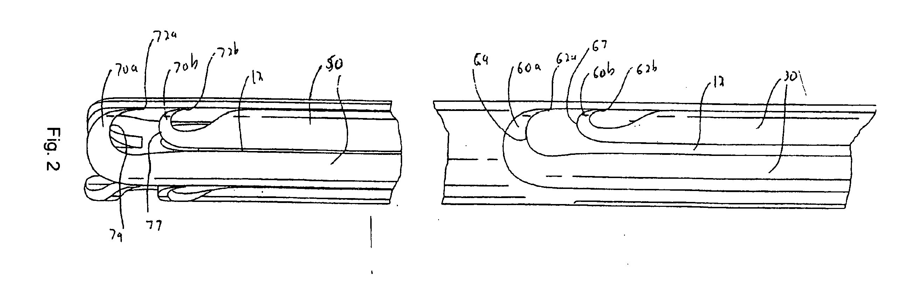

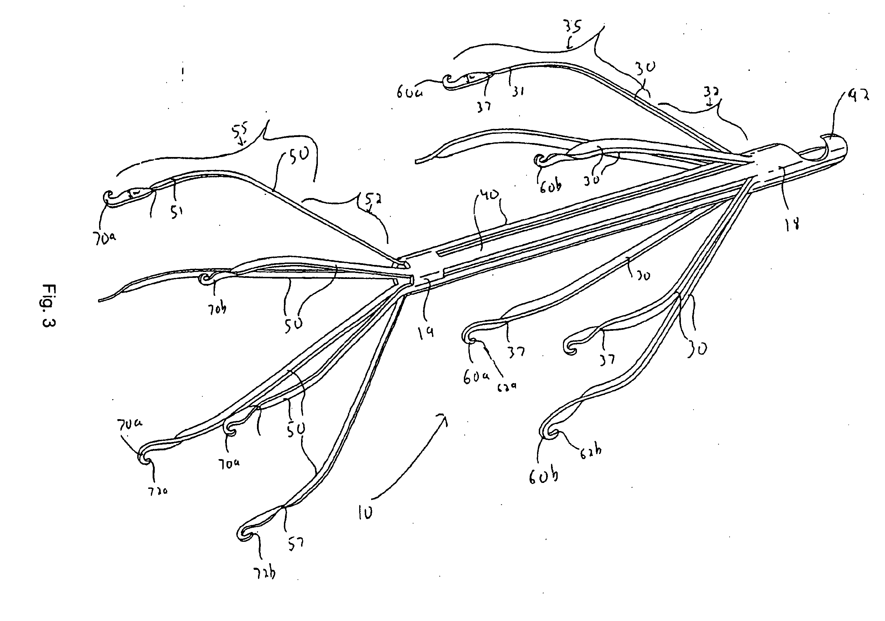

Turning now to details of the filter of a first embodiment of the present invention and with initial reference to Figures 1 and 2, the filter is designated generally by reference numeral 10 and is shown in a collapsed configuration for delivery. Filter 10 is preferably formed from a single tube 11. In a preferred embodiment, the filter 10 is composed of shape memory material, such as Nitinol, a nickel titanium alloy, or elgiloy, however, other materials such as stainless steel are also contemplated. A plurality of cutouts 12 are formed in the filter 10, preferably by laser cutting although other techniques are contemplated. In the illustrated embodiment, six elongated cutouts are formed in the first region 15 and in the second region 17, creating two pairs of six strips or struts 30, 50 of substantially uniform width separated by the cutouts 12. The first set of struts 30 thus extends from tubular portion 18 and the second set of struts 50 extends from tubular portion 19. Longitudinal struts 40 extend between tubular portions 18 and 19, thus connecting the two sets of struts (see Figure 3).

The collapsed configuration of filter 10 reduces the overall profile to facilitate delivery to the site. The diameter of filter 10 in the collapsed configuration is represented by reference D1 and preferably is about 2mm and more preferably about 1.7mm. Other dimensions are also contemplated. The filter is thus preferably dimensioned for insertion through a 6 French delivery system or 6 French catheter. The diameter or transverse dimensions of the filter in the expanded placement configurations is greater than the diameter or transverse dimension D1 in the collapsed (delivery) configuration.

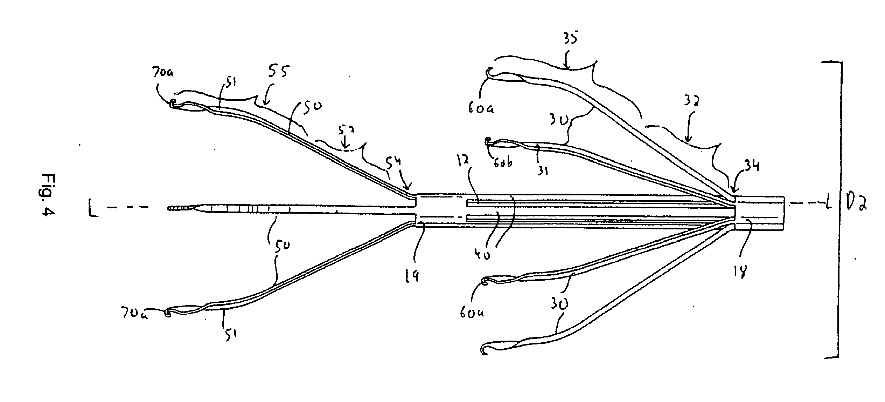

Figures 3-5 illustrate the expanded placement configuration of the filter 10. As noted above, filter 10 has a first set of struts 30 and a second set of struts 50, each forming bell-shaped regions in the expanded configuration. The struts 30 and 50 at one end each have a filtering region 32, 52 having a converging region 34, 54, respectively. At the opposing end, the struts 30, 50 each have a flared region 35, 55. In larger vessels, the filter can expand to a diameter or transverse dimension D2 shown in Figure 4. In smaller vessels, the filter expands to a smaller diameter than in larger vessels. Diameters (or transverse dimensions) preferably range from about 18 mm to about 32mm, depending on the internal diameter of the vessel wall as will be explained in more detail below. Other dimensions are also contemplated.

By providing two sets of struts, two levels of filtration are provided as the second set of struts acts as a backup. To enhance the backup function, the second set of struts is preferably offset with respect to the first set of struts. That is, each strut 50 is about 30 degrees out of phase (longitudinal alignment) from a corresponding strut 30. Thus, preferably, the elongated struts 30 and 50 are identical except for their radial offset. Although shown about 30 degrees out of phase, other spacing is also contemplated.

Struts 30 are spaced apart as shown and extend at an angle away from the longitudinal axis L of filter 10 in region 35 to provide a flare. Preferably, this angle or taper is about 10 degrees, although other dimensions are contemplated. In the filtering region 32, beginning at an intermediate portion of the filter (the transition between the regions 35, 32) the struts 30 extend inwardly to the longitudinal axis at an angle to the respective tubular portion 18 thereby forming an angle with the longitudinal axis. That is, filtering section 32 extends from the flared region toward the central longitudinal axis L of the filter 10 and converges at portion 34 into tubular portion 18. For clarity, not all of these sections of each strut 30, 50 are labeled in the drawings, it being understood that the non-labeled struts have the same configurations.

Struts 50, in the illustrated embodiment, are identical to struts 30. That is, struts 50 are spaced apart as shown and extend at an angle preferably about 10 degrees (other dimensions are contemplated) away from the longitudinal axis L of filter 10 to provide a flare. In the filtering region 52, beginning at an intermediate portion, the struts 50 extend inwardly toward the longitudinal axis at an angle to the tubular portion 19, thereby forming an angle with the longitudinal axis.

In the illustrated embodiment, when expanded, the six struts 50 and the six struts 30 are shown spaced approximately 60 degrees apart. It is also contemplated that a fewer or greater number of struts could be provided and spacing other than 60 degrees be provided.

In the expanded placement configuration, a portion of the each elongated strut 30 and 50 has an outer surface 31, 51 respectively, for engagement with the vessel wall to retain the filter 10 in position in the vessel. This region is angled with respect to the longitudinal axis. The outer surface 31, 51 of struts 30, 50 could be roughened to enhance engagement. Alternatively, a plurality of cutouts, atraumatic tabs, barbs or other penetrating members (not shown) can extend from the outer surface 31, 51 of one or more of the struts to engage the vessel wall to retain the filter.

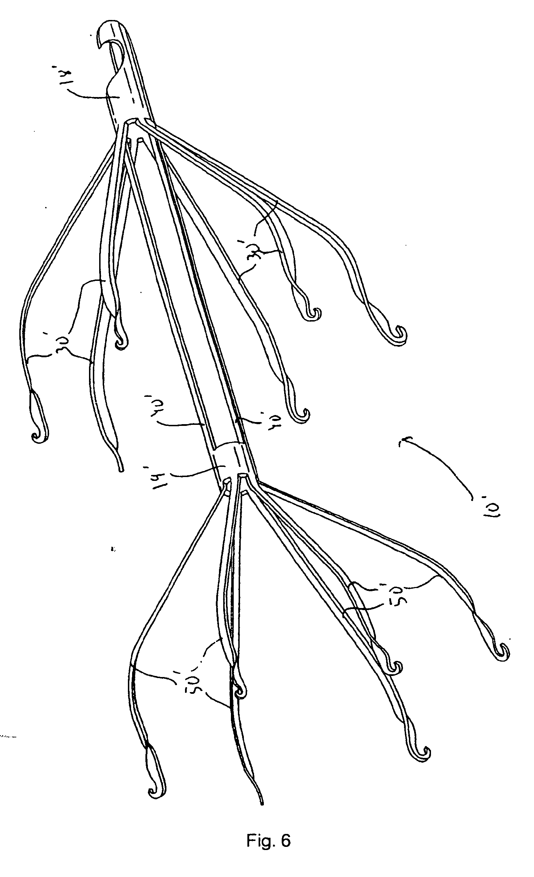

As can be appreciated, the tubular portion 11 connects the struts 30 and 50. As shown in Figure 3, since six cutouts are formed, six elongated struts 40 extend from tubular portion 18 to tubular portion 19, thereby connecting the regions 15 and 17. In the alternate embodiment of Figure 6, some of the longitudinal struts have been removed so that only two longitudinal struts 40' extend between tubular portions 18' and 19'. This reduces the amount of material which is placed in the body. It is also contemplated that a fewer or greater number of longitudinal struts could be provided in these embodiments. Otherwise, filter 10' is identical to filter 10 and has been labeled with "prime" designations to illustrate the corresponding parts with Figure 3. For clarity, not all the parts have been labeled.

Referring back to Figures 3 and 4, each of the struts 30, 50 terminates in a hook 60, 70, respectively, which extend substantially perpendicular from the strut. This arrangement is achieved by torquing the struts 30, 50 at the respective region 37, 57 (or along an extended length of the strut) so the hook portions bend out of the plane. The hooks 60, 70 of filter 10 lie in the plane of the connecting end strut region 37, 57 aligned with the width surface "w" of the region. The hooks can alternatively be formed or placed on fewer than all the struts.

In the illustrated embodiment, the hooks of filter 10 in each region 15, 17 are of two different sizes. More specifically, in first region 15, a first set of hooks 60a is larger than a second set of hooks 60b. Preferably, when formed in a laser cut tube, hooks 60a are formed so that they occupy a region equivalent to the transverse dimension of two adjacent struts. For example, in the collapsed configuration, hook 60a occupies a region (dimension) of four connecting struts while smaller hook 60b would only occupy the region (dimension) of two connecting struts. Smaller hooks 60b are spaced axially inwardly with respect to larger hooks 60a to minimize the collapsed profile (transverse dimension) of the filter when collapsed for insertion. In this preferred embodiment, smaller hooks 60b occupy the space created by the larger hooks 60a so they can be considered as nesting within larger hooks 60a (see Figure 2). Stated another way, each hook 60b has an outer surface 67 which conforms (follows the contour) to an inner surface 69 of a hook 60a. The penetrating tips 62a, 62b of hooks 60a, 60b, respectively, penetrate the tissue to retain the filter, preferably temporarily. Hooks 70a, 70b of region 17 are identical to hooks 60a, 60b, respectively, having outer surface 77, inner surface 79, and penetrating tips 72a, 72b

The hooks or other vessel engaging structure can be placed on both sets of struts 30, 50 or alternatively be placed only on struts 30 or only on struts 50 or placed on fewer than all the struts of the particular set of struts.

A recess or cutout can also be provided at the tubular end portion to receive a snare or other device for removal. In the preferred embodiment, a hook 92 at tubular portion 18 is illustrated and is configured to receive a snare.

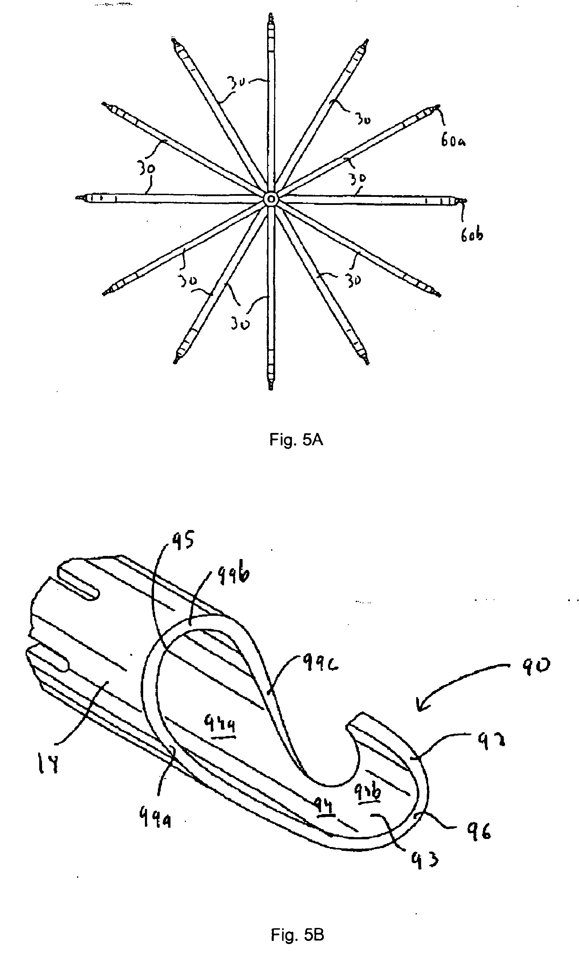

Hook 90 has a curved hook 92 at the proximalmost end. Hook 92 is configured to receive a retrieval snare or other retrieval device. A portion of the wall of the hook 90 is cut out to expose the annular interior surface 94 (see Figure 5B). That is, being formed from a laser cut tube, a wall portion is removed to expose curved inner wall surface 94. This annular interior surface 94 extends from radiused region 95 to proximalmost edge 96. The interior surface 94, for ease of explanation, can be considered to have an interior surface 94a at the radiused region 95 and an interior surface 94b at the hook 92. The interior surface 94b accommodates a portion of a tubular snare sheath. That is, the outer wall of the snare sheath (tube) can partially fit within the cut out region 93. This enhances removal as the snare pulls the filter hook into collinear arrangement with the sheath tube. The radiused region 95, spaced axially (distal) from the hook 92, includes a radiused or curved edge defined by radiused side walls 99a, 99c and a top wall 99b. The angled side walls 99a, 99c form camming surfaces to direct the hook 90 and filter into the retrieval sheath.

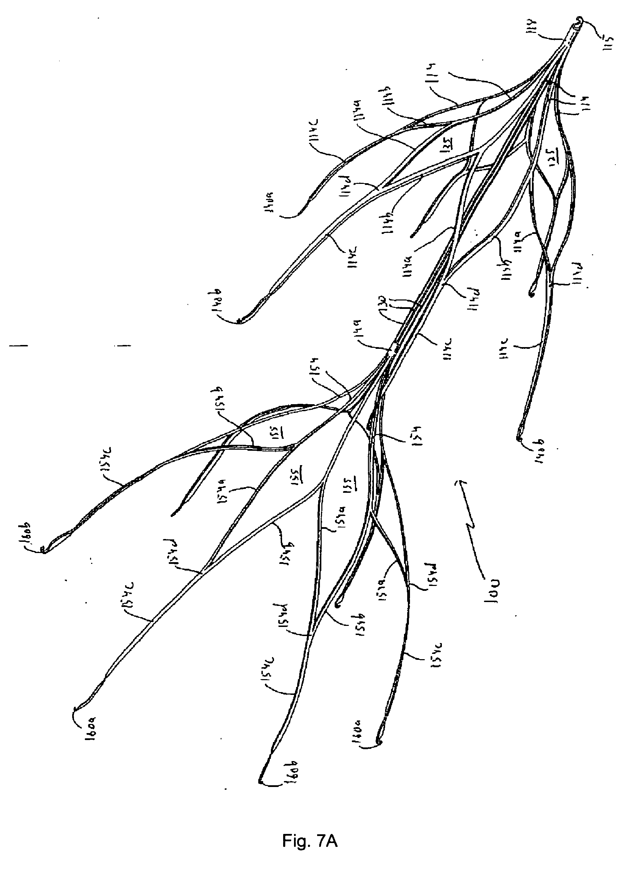

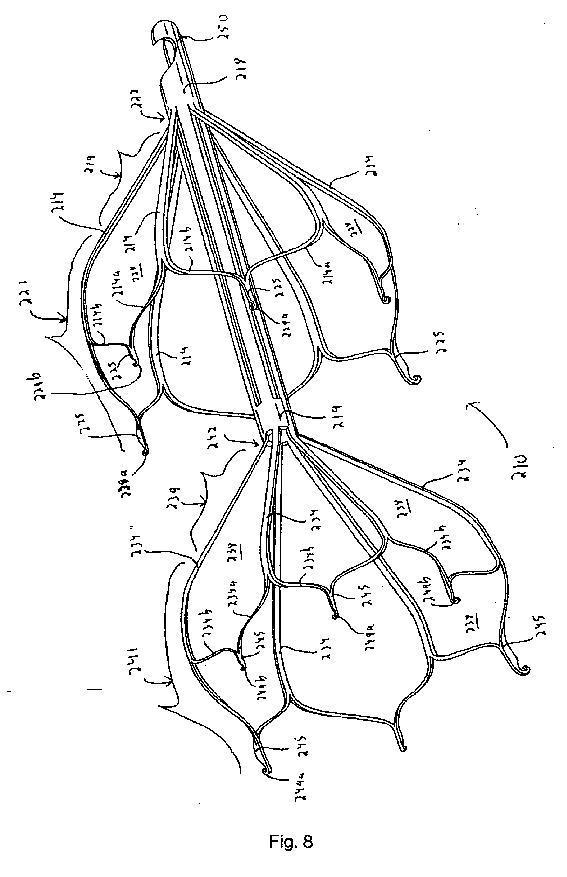

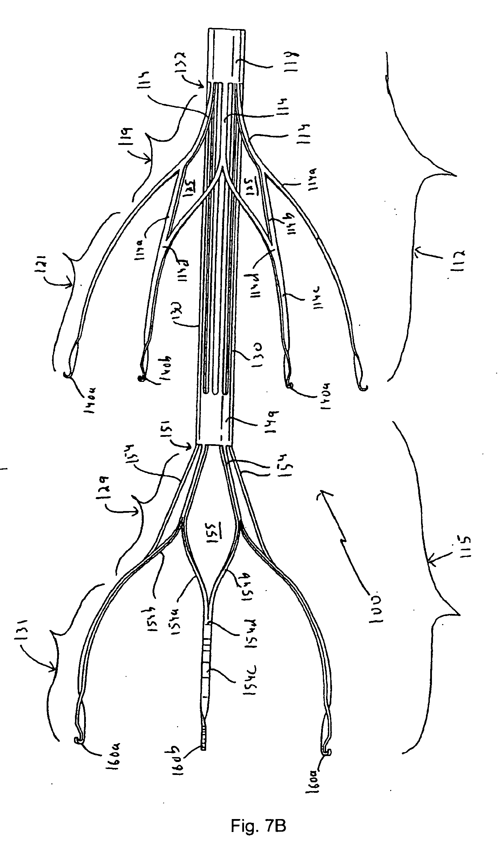

Figures 7A and 7B illustrate an alternate embodiment of the filter of the present invention. In this embodiment, the struts of filter 100 are interconnected at the filtering region. This creates closed geometric shapes at the filtering region to enhance the clot capturing capability of the filter. Also, by providing the interconnection more forward (downstream) in the filter, i.e. in the filtering region (filtration zone), rather than in the mounting region as described below with respect to the embodiment of Figure 8, linear movement of the filter is facilitated to enhance removal of the filter.

Bell-shaped filter 100 has a filtering region (portion/section) 119 and a flared anchoring (mounting) region (portion/section) 121 in first region 112 and a filtering region (portion/section) 129 and anchoring (mounting) region (portion/section) 131 in second region 115. The mounting regions 121, 131, are of greater transverse dimension than the respective filtering regions 119, 129. Flared regions 121 and 131 are preferably at an angle of about 10 degrees with respect to the longitudinal axis of the filter, although other angles are contemplated. In these flared regions 121, 131 the transverse dimension increases towards the anchoring end of the filter 100 so that as in the other embodiments disclosed herein, the end of the filter at regions 119, 129 have a smaller transverse dimension than at the opposing end at the respective flared regions 121, 131. The filtering region 119 extends from the flared region 121 toward the longitudinal axis of the filter 100 and converges at portion 132 into tubular portion 118. The filtering region 129 extends from flared region 131 toward the longitudinal axis and converges at portion 151 into tubular portion 149. Longitudinally extending struts 130 connect the two sets of axially spaced struts 114, 154. Providing fewer longitudinal struts 130 is also contemplated.

Filtering region 119 preferably has six struts 114 curving outwardly from tubular portion 118. Each filter strut or strut portion 114 extends radially from tubular portion 118 and divides into two connecting filter struts or strut portions 114a, 114b (preferably of equal width) that angle way from each other (in different directions) to extend to the connecting strut portion of an adjacent strut 114. Thus, connecting strut portion 114a of one strut 114 interconnects with the connecting strut portion 114b of an adjacent strut at joining or connecting region 114d. This forms closed geometric shapes 125, preferably substantially diamond shaped in configuration. For clarity, not all of the identical parts are labeled in the drawing. In the illustrated embodiment, preferably six struts are provided forming twelve interconnecting struts, however a different number of struts and closed geometric shapes can be provided. Also, fewer than all of the struts could be interconnected. Although preferably the struts 114 (and 154 described below) divide into connecting struts 114a, 114b of half the width of the undivided strut 114, the struts can bifurcate to form connecting struts of other dimensions.

After convergence of strut portions 114a, 114b at joining region 114d, it transitions into elongated mounting strut portions 114c which form flared mounting or anchoring region 121. The length of the strut portions 114c in the anchoring region 121 can vary, with increased/decreased length increasing the flexibility/rigidity of the struts. The thickness of the strut portions can also vary to affect flexibility/rigidity.

Interconnecting struts are preferably also provided on struts 154 in second region 115. The struts 154 and interconnecting struts or strut portions 154a, 154b in the illustrated embodiment are identical to struts 114 and interconnecting struts 114a, 114b of first region 112. Thus, they join at region 154d, form closed geometric shapes 155 and have mounting strut portions 154c extending from joining region 154d.

Preferably, the strut portions 114c, 154c terminate in hooks 140a, 140b and 160a, 160b similar to hooks 60a, 60b and 70a, 70b, respectively, of Figure 3. That is, the hooks lie in the plane of the respective struts 114c, 154c and hooks 140a are larger than hooks 140b, and hooks 160a are larger than hooks 160b. The larger hooks are formed so they occupy a region equivalent to the transverse dimension of two adjacent struts. Smaller hooks 140b, 160b nest within larger hooks 140a, 160a as described above in conjunction with hooks 60a, 60b and 70a, 70b. Note that smaller hooks 140b, 160b are spaced axially (inwardly) of hooks 140a, 160a, as well as spaced axially with respect to each other (as in hooks 60b and 70b). Other hook designs could alternatively be provided.

Although interconnecting struts are shown on both sets of filter struts, alternatively they can be provided on only one set. Also, alternatively not all struts are interconnected.

Filter 100 can also preferably have a retrieval hook, such as hook 115 formed in tubular portion 118 which is identical to hook 92 of Figure 1.

Figure 8 illustrates an alternate embodiment of the filter, designated by reference numeral 210. Filter 210, having a first set of struts 214 and a second set of struts 234, is similar to filter 10 except for anchoring regions 221, 241. That is, like filter 10, filter 210 has two filtering regions 219, 239 which extend from the flared anchoring regions 221, 241, and extend toward the central longitudinal axis of the filter 210 and converge at portions 222, 242, into tubular portions 218, 219, respectively. Filter 210 preferably has a retrieval hook, such as hook 250, which is identical to hook 92 of Figure 1, although other hooks are contemplated. For clarity, not all of these sections of each strut 214, 234 are labeled in the drawing, it being understood that the non-labeled struts have the same configurations. The flared regions, as in filter 10, are each of an angle preferably about 10°, although other angles are contemplated.

The region 225 of filter 210 where the struts 214 interconnect (join) and the region 245 where struts 234 interconnect differ from filter 10. In filter 210, the struts 214 are interconnected by connecting strut portions 214a, 214b that curve outwardly away from the central axis and then inwardly toward each other. The connecting struts are joined to connecting struts of adjacent struts at region 225. Thus, closed geometric shapes 228 are formed as shown. Six such closed geometric shapes 228 are preferably formed, each connecting adjacent struts, although fewer closed shapes are contemplated if fewer than all the struts are interconnected.

Thus, stated in other words, each strut 214 bifurcates or divides into two connecting strut portions 214a, 214b which initially extend outwardly from each other. As each strut extends outwardly, the strut portion 214a joins the strut portion 214b of an adjacent strut at region 225. After this joined region 225, the strut portions 214a and 214b which emanate from the same strut extend into hook regions and terminate in hooks 229a, 229b, similar to hooks 60a, 60b of Figure 3.

Similarity, each strut 234 bifurcates or divides into connecting strut portions 234a, 234b forming closed geometric shapes 238. The connecting struts 234a, 234b are joined at region 245 and extend into hook regions, terminating in hooks 249a, 249b similar to hooks 229a, 229b.

Although shown divided into equally dimensioned struts, as described above with respect to the Figure 7 embodiment, the struts can bifurcate into connecting struts of varying dimension.

In the placement (expanded) configuration, each filter of the present invention moves towards its memorized position and the extent it returns to its fully memorized position will be dependent on the size of the vessel in which the filter is inserted. (The larger the vessel, the closer the filter comes to returning to its fully memorized position.)

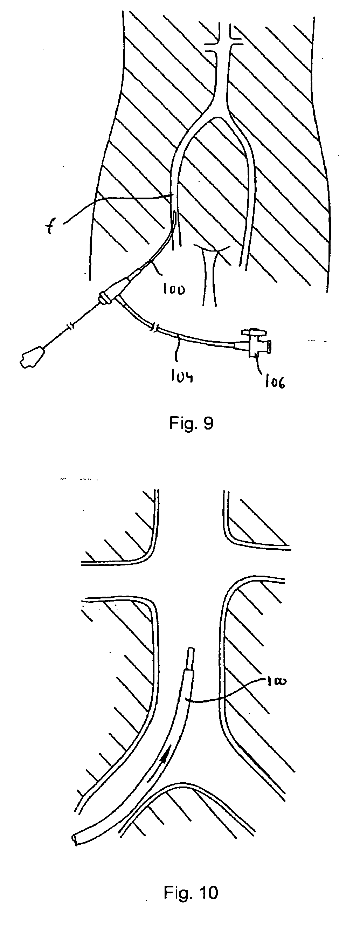

To enable movement between an expanded and collapsed configuration, the filter tube of the embodiments described herein is preferably made of shape memory metal material, such as Nitinol, a nickel titanium alloy. The memorized configuration of the filter 10 is shown in Figure 3. To facilitate passage of the filter 10 through the lumen of the delivery sheath 100 (shown in Figure 9 in conjunction with the method of insertion) and into the vessel, cold saline is injected into the delivery sheath or catheter 100 and around the filter 10 in its collapsed position within the delivery sheath 100. This shape memory material characteristically exhibits rigidity in the austenitic state and more flexibility in the martensitic state. The cold saline maintains the temperature dependent filter 10 in a relatively softer condition as it is in the martensitic state within the sheath. This facilitates the exit of filter 10 from the sheath 100 as frictional contact between the filter 10 and the inner surface of the sheath would otherwise occur if the filter was maintained in a rigid, i.e. austenitic, condition.

Once ejected from the delivery sheath or catheter 100, the filter is no longer cooled and is exposed to the warmer body temperature, which causes the filter 10 to return towards its austenitic memorized configuration. Filters 10, 100 and 210 as well as filter 300, 400, 500 and 500', operate in a similar manner.

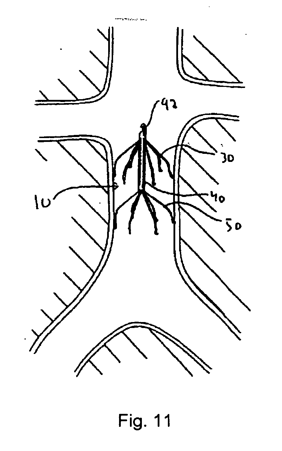

Figs. 9-11 illustrate delivery and placement of the filter 10, by way of example, in the inferior vena cava. Delivery catheter 100 is inserted through the femoral vein "f" and advanced through the iliac arteries into the inferior vena cava. Delivery catheter would be withdrawn once the tip of the sheath is adjacent the structure so that withdrawal of the sheath would place the filter in the desired location of Fig. 11. Tubing 104 and valve assembly 106 enable saline injection. Delivery catheter 100 is withdrawn to enable filter 10 to be warmed by body temperature to transition to the expanded placement configuration. The other filters described herein could be inserted in the same manner. This enables blood clots or other particles to be directed to the center of the filter section by the angled struts. Thus the direction of insertion, e.g. upstream or downstream direction, will determine how the filter is to be positioned in the delivery catheter.

Figures 12-22 illustrate alternate embodiments of the filter of the present invention having a single filtering portion and two mounting portions.

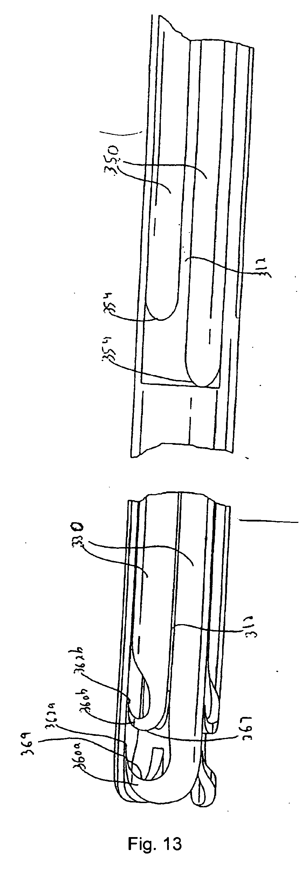

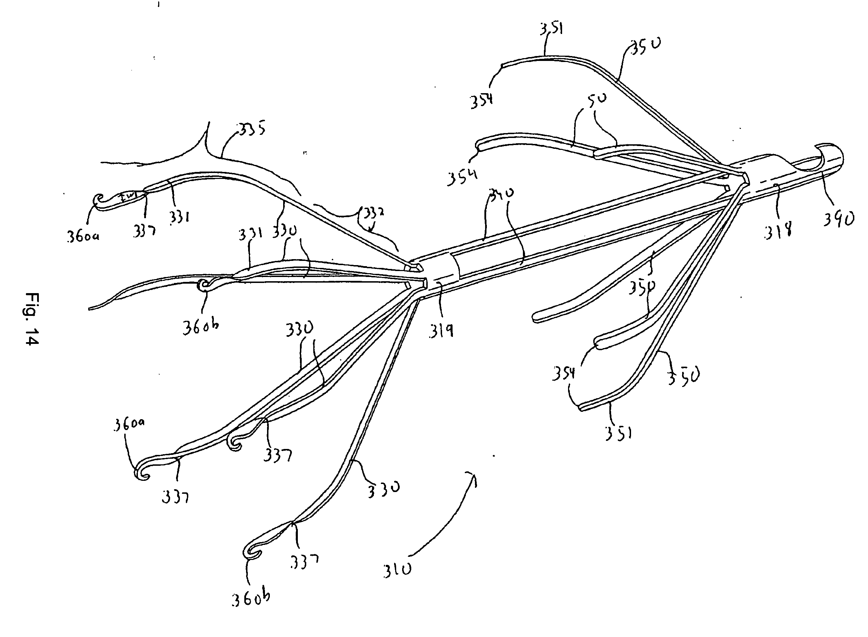

Turning first to the embodiment of Figures 12-16, and with initial reference to Figures 12 and 13, the filter is designated generally by reference numeral 310 and is shown in a collapsed configuration for delivery. Filter 310 is preferably formed from a single tube 311. In a preferred embodiment, the filter 310 is composed of shape memory material, such as Nitinol, a nickel titanium alloy, or elgiloy; however, other materials such as stainless steel are also contemplated. The memorized configuration of the filter 310 is shown in Figure 14. A plurality of cutouts 312 are formed in the filter 310, preferably by laser cutting although other techniques are contemplated. In the illustrated embodiment, six elongated cutouts are formed in the first region 315 and in the second region 317, creating two pairs of six strips or struts 330, 350, respectively, separated by the cutouts 312. Preferably the struts in each set are of substantially uniform width. The second set of struts 350, used for anchoring or mounting the filter, thus extends from tubular portion 318 and the first set of struts 330, used for filtering and additional anchoring, extends from tubular portion 318. Longitudinal struts 340 extend between tubular portions 318 and 319, thus connecting the two sets of struts (see Figure 14).

The collapsed configuration of filter 310 reduces the overall profile to facilitate delivery to the site. The diameter of filter 310 in the collapsed configuration is represented by reference P1 and preferably is about 2mm and more preferably about 1.7mm. Other dimensions are also contemplated. The filter is thus preferably dimensioned for insertion through a 6 French delivery system or 6 French catheter. The diameter or transverse dimensions of the filter in the expanded placement configurations is greater than the diameter or transverse dimension P1 in the collapsed (delivery) configuration.

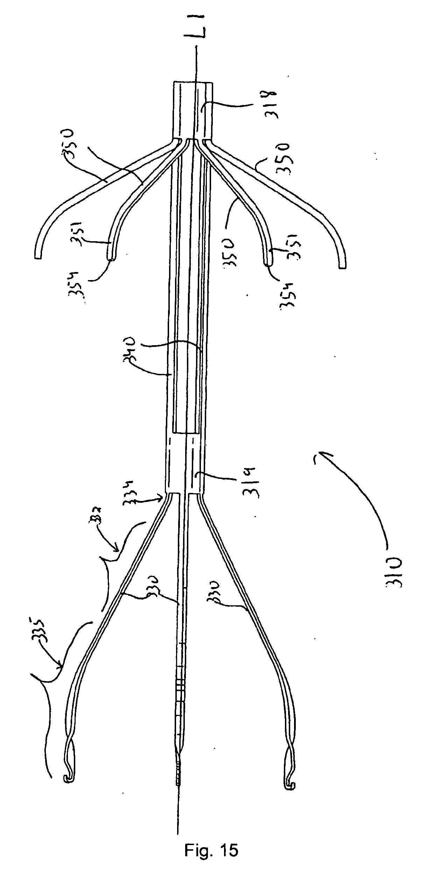

Figures 14-16 illustrate the expanded placement configuration of the filter 310. As noted above, filter 310 has a first set of struts 330 forming a bell-shaped region in the expanded configuration. The struts 330 have a filtering region 332 having a converging region 334. At the opposing end, the struts 330 have a flared region 335. In larger vessels, the filter can expand to a diameter or transverse dimension shown in Figure 15. In smaller vessels, the filter expands to a smaller diameter than in larger vessels. Diameters (or transverse dimensions) preferably range from about 18 mm to about 32 mm, depending on the internal diameter of the vessel wall as will be explained in more detail below. Other dimensions are also contemplated.

Struts 330 are spaced apart as shown and extend at an angle away from the longitudinal axis L of filter 310 in region 335 to provide a flare. Preferably, this angle or taper is about 10 degrees, although other dimensions are contemplated. In the filtering region 332, beginning at an intermediate portion of the filter (the transition between the regions 335, 332) the struts 330 extend inwardly to the longitudinal axis at an angle to the respective tubular portion 319 thereby forming an angle with the longitudinal axis. That is, filtering section 332 extends from the flared region toward the central longitudinal axis L1 of the filter 310 and converges at portion 334 into tubular portion 319. For clarity, not all of these sections of each strut 330 are labeled in the drawings, it being understood that the non-labeled struts have the same configurations.

Struts 350 are spaced apart as shown and extend at an angle preferably about 60 degrees (other dimensions are contemplated) away from the longitudinal axis L1 of filter 310. The struts 350 extend outwardly from the longitudinal axis at an angle to the tubular portion 318, thereby forming an angle with the longitudinal axis, and then curve outwardly at a lesser angle to provide mounting portions. The struts 350 are preferably flared. The struts 350 can have an elongated mounting surface to engage the vessel wall.

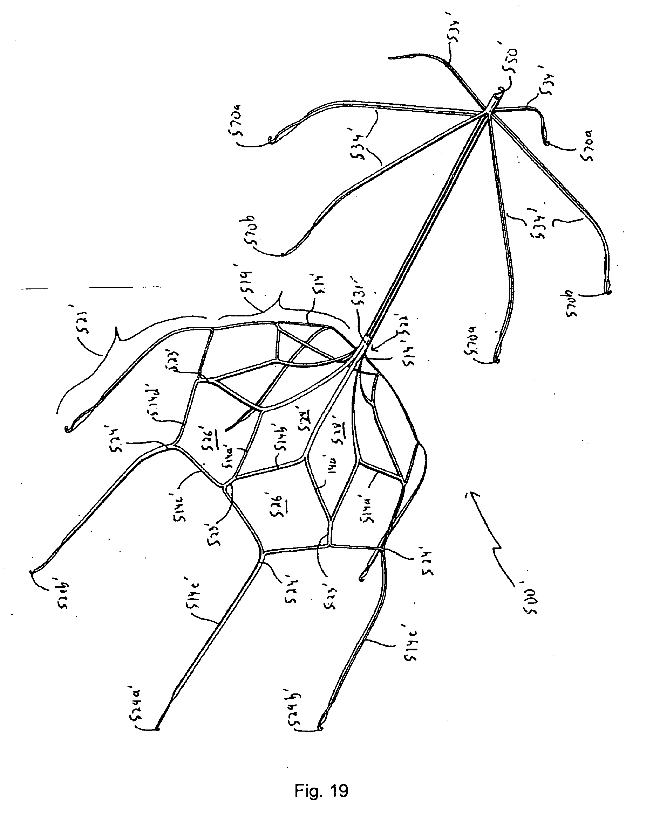

In the embodiment of Figure 14, the struts 350 terminate in blunt tips 354. In this embodiment, the radial force of the struts 350 helps to retain the filter and helps prevent tilting and promote self-centering. In an alternate embodiment, the struts 350 can terminate in vessel engaging hooks. These are described below for example in the embodiment of Figure 19, although such hooks can be placed on any of the filter embodiments described herein to enhance retention.

In the illustrated embodiment, when expanded, the six struts 350 and the six struts 330 are shown spaced approximately 60 degrees apart. It is also contemplated that a fewer or greater number of struts could be provided and spacing other than 60 degrees be provided. The struts 350 can also be radially offset from the struts 330, e.g., by 30 degrees. It is also contemplated that struts 350 can have a larger transverse dimension than struts 330.

In the expanded placement configuration, a portion of each elongated strut 330 and 350 has an outer surface 331, 351 respectively, for engagement with the vessel wall to help retain the filter 10 in position in the vessel. This region is angled with respect to the longitudinal axis. The outer surface 331,351 of struts 330, 350 could be roughened to enhance engagement. Alternatively, a plurality of cutouts, atraumatic tabs, barbs or other penetrating members (not shown) can extend from the outer surface of one or more of the struts to engage the vessel wall to retain the filter.

As can be appreciated, the tubular portion 311 connects the struts 330 and 350. As shown in Figure 14, two elongated struts 340 extend from tubular portion 318 to tubular portion 19, thereby connecting the regions 315 and 317. This can be achieved by removing some of the longitudinal struts during manufacturing. This reduces the amount of material, which is placed in the body. Alternatively, six longitudinal struts formed by the six cutouts could connect the regions 315 or 317, or a different number of struts could be provided.

With continued reference to Figures 14 and 15, each of the struts 330 terminates in a hook 360a, 360b which extends substantially perpendicular from the strut. This arrangement is achieved by torquing the struts 330 at the respective region 337 (or along an extended length of the strut) so the hook portions bend out of the plane. The hooks 360a, 360b of filter 310 lie in the plane of the end of the strut region, aligned with the width surface "w1" of the region. The hooks can alternatively be formed or placed on fewer than all of the struts.

In the illustrated embodiment, the hooks of filter 310 in region 317 are of two different sizes as in the hooks 70a, 70b described above. That is, a first set of hooks 360a is larger than a second set of hooks 360b and are preferably formed in a laser cut tube. Hooks 360a, 360b are preferably identical to hooks 70a, 70b of the embodiment of Figure 1, e.g., hook 360a occupies a region of two struts, smaller hooks 360b are spaced axially inwardly with respect to larger hooks 360a and nest within larger hooks 360a (see Figure 13) as each hook 360b has an outer surface 367 which conforms to an inner surface 369 of a hook 360a. The penetrating tips 362a, 362b of hooks 360a, 360b, respectively, penetrate the tissue to retain the filter, preferably temporarily.

It should be appreciated that the hooks or other vessel engaging structure can be placed on both sets of struts 330, 350 or alternatively be placed only on struts 330 or only on struts 350 or placed on fewer than all the struts of the particular set of struts.

A recess or cutout can be provided at the tubular end portion 318 to receive a snare or other device for removal. In the preferred embodiment, a hook 392 at tubular portion 318 is illustrated and is configured to receive a snare.

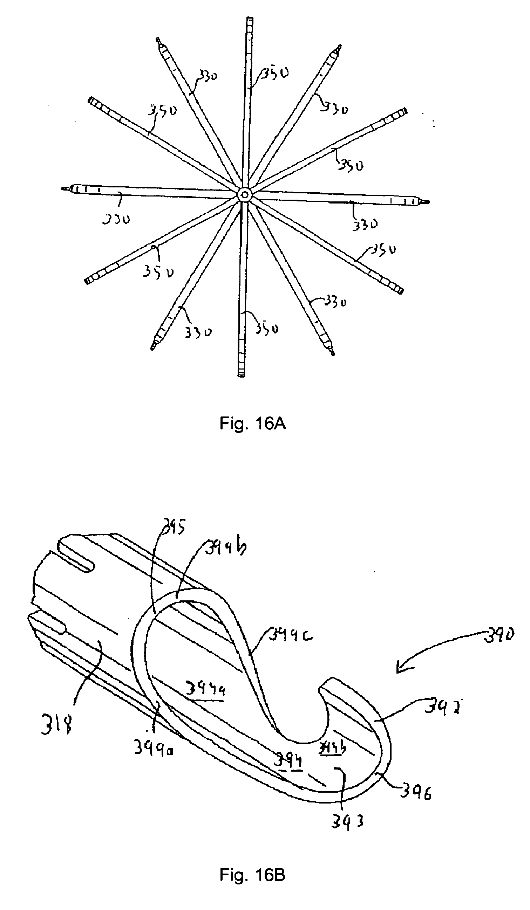

Hook 390 in a preferred embodiment is identical to hook 90 of Figure 1. That is, it has a curved hook 392 to receive a retrieval snare or other retrieval device of an annular interior surface 394 (see Figure 16B), extending from radiused region 395 to proximalmost edge 396 and having an interior surface 394a at the radiused region 395 and an interior surface 394b at the hook 392. The outer wall of the snare sheath (tube) can partially fit within the cut out region 393 to enhance removal as described above with respect to hook 90 into collinear arrangement with the sheath tube. The radiused side walls 399a, 399c adjacent top wall 399b form camming surfaces like walls 99a, 99c of hook 90.

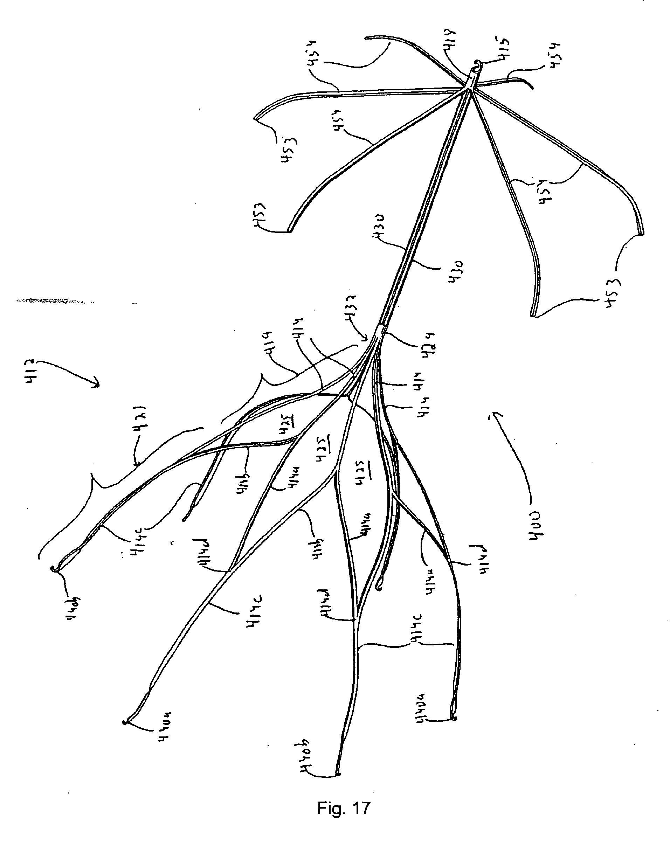

Figure 17 illustrates an alternate embodiment of the filter of the present invention. In this embodiment, the struts of filter 400 are interconnected at the filtering region. This creates closed geometric shapes at the filtering region to enhance the clot capturing capability of the filter. Also, by providing the interconnection more forward (downstream) within the first region 412, i.e. in the filtering region (filtration zone), linear movement of the filter is facilitated to enhance removal of the filter.

Filter 400 has a filtering region (portion/section) 419 and a flared anchoring (mounting) region (portion/section) 421 in first region 412, forming a bell shape. The mounting region 421 is of greater transverse dimension than the respective filtering region 419. Flared region 421 is preferably at an angle of about 10 degrees with respect to the longitudinal axis of the filter, although other angles are contemplated. In this flared region 421, the transverse dimension increases towards the anchoring end of the region 412 so that as in the other embodiments disclosed herein, the end of the filter at region 419 has a smaller transverse dimension than at the opposing end at the respective flared regions 421. The filtering region 419 extends from the flared region 421 toward the longitudinal axis of the filter 400 and converges at portion 432 into tubular portion 429. Longitudinally extending struts 430 connect the two sets of axially spaced struts 414, 454. Providing fewer longitudinal struts 430 is also contemplated.

Filtering region 419 preferably has six struts 414 curving outwardly from tubular portion 429. Each filter strut or strut portion 414 extends radially from tubular portion 429 and divides into two connecting filter struts or strut portions 414a, 414b (preferably of equal width) that angle away from each other (in different directions) to extend to the connecting strut portion of an adjacent strut 414. Thus, connecting strut portion 414a of one strut 414 interconnects with the connecting strut portion 414b of an adjacent strut at joining or connecting region 414d. This forms closed geometric shapes 425, preferably substantially diamond shaped in configuration. For clarity, not all of the identical parts are labeled in the drawing. In the illustrated embodiment, preferably six struts are provided forming twelve interconnecting struts, however a different number of struts and closed geometric shapes can be provided. Also, fewer than all of the struts could be interconnected. Also, although preferably the struts 414 divide into connecting struts 414a, 414b of half the width of the undivided strut 414, the struts can bifurcate to form connecting struts of other dimensions. It is also contemplated that fewer than all of the struts can be interconnected.

After convergence of strut portions 414a, 414b at joining region 414d, it transitions into elongated mounting strut portions 414c which form flared mounting or anchoring region 421. The length of the strut portions 414c in the anchoring region 421 can vary, with increased/decreased length increasing the flexibility/rigidity of the struts. The thickness of the strut portions can also vary to affect flexibility/rigidity.

Struts 454 of filter 400 are preferably identical to struts 350 of Figure 14. Thus, struts 454 extend outwardly from the longitudinal axis at an angle to the tubular portion 418 thereby forming an angle with the longitudinal axis, and then curve outwardly at a lesser angle to provide mounting portions. The struts 454 are preferably flared and have blunt ends 453.

Preferably, the strut portions 414c terminate in hooks 440a, 440b identical to hooks 360a, 360b of Figure 14. That is, the hooks lie in the plane of the respective struts 414c and hooks 440a are larger than hooks 440b. The larger hooks are formed so they occupy a region equivalent to the transverse dimension of two adjacent struts. Smaller hooks 440b nest within larger hooks 440a as described above in conjunction with hooks 70a, 70b and 360a, 360b. Note that smaller hooks 440b are spaced axially (inwardly) of hooks 440a as well as spaced axially with respect to each other as in hooks 160b. Other hook designs could alternatively be provided. Struts 454 could also optionally terminate in hooks similar to hooks 440a and/or 440b (or other vessel engaging structure) instead of blunt tip 453 as in the embodiment of Figure 17.

Filter 400 can also preferably have a retrieval hook, such as hook 415 formed in tubular portion 418 which is identical to hook 390 of Figure 12.

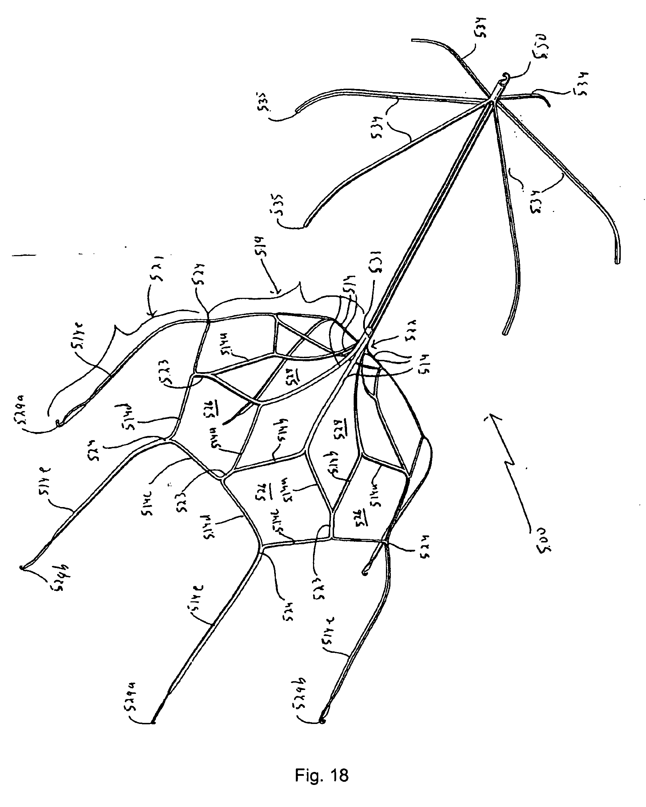

Figure 18 illustrates another alternate embodiment of the filter, designated by reference numeral 500. Filter 500, having a first set of struts 514 and a second set of struts 534, is similar to filter 110 except for filtering region 519. That is, like filter 110, filter 500 has a filtering region 519, which extends from the flared anchoring region 521 and extends toward the central longitudinal axis of the filter 500, with struts 514 converging at portion 522 into tubular portion 531. The flared region, as in filter 110, is at an angle preferably about 10°, although other angles are contemplated. Struts 534 are identical to struts 454 of Figure 17 and preferably have blunt tips 535. For clarity, not all of these sections of each strut 514, 534 are labeled in the drawing, it being understood that the non-labeled struts have the same configurations. Filter 500 preferably has a retrieval hook, such as hook 550, which is identical to hook 390 of Figure 12, although other hooks are contemplated.

The region 519 of filter 500 where the struts 514 interconnect (join) differs from filter 400. In filter 500, the struts 514 bifurcate into connecting strut portions 514a, 514b that angle away from each other. The connecting stru ts are joined to connecting struts of adjacent struts at joining region 523. Thus, closed geometric shapes 528 are formed as shown. Six such closed geometric shapes 528 are preferably formed, each connecting adjacent struts, although fewer closed shapes are contemplated if fewer than all the struts are interconnected. Thus, stated in other words, as in the embodiment of Figure 17, each strut 514 bifurcates or divides into two connecting strut portions 514a, 514b which initially extend outwardly from each other. As each strut extends outwardly, the strut portion 514a joins the strut portion 514b of an adjacent strut at region 523. However, after convergence of strut portions 514c, 514d at joining region 523, it bifurcates into connecting strut or strut portions 514c, 514d that angle away from each other. Connecting struts 514c, 514d of adjacent struts are joined at region 524, thus forming six closed geometric shapes 526. Strut portions 514c extend from joined region 524 and transition into hook regions and terminate in hooks 529a, 529b, preferably identical to hooks 360a, 360b of Figure 14.

Although shown divided into equally dimensioned struts, as with the other embodiments described herein, the struts can bifurcate into connecting struts of varying dimension.

In the alternate embodiment of Figure 19, filter 500' is identical to filter 500 of Figure 18 except for the provision of hooks 570a, 570b on struts 534'. These hooks 570a, 570b provide additional retention of the filter 500'. The hooks 570a, 570b are preferably identical to hooks 529a, 529b, respectively, of Figure 18. Since the other features are identical to filter 500, corresponding parts are provided with "prime" designations. For clarity, not all parts are labeled.

Note the designations of longitudinal, angled, curved, bowed, connected, joined connecting strut, interconnected, etc. in the illustrated embodiments described herein refer to the same integral strut and are divided into such regions for ease of understanding.

To facilitate passage of the filter 310 through the lumen of the delivery sheath 700 (shown in Figure 9 in conjunction with the method of insertion) and into the vessel, cold saline is injected into the delivery sheath or catheter 700 and around the filter 310 in its collapsed position within the delivery sheath 700 as described above with respect to filter 10. The cold saline maintains the temperature dependent filter 310 in a relatively softer condition as it is in the martensitic state within the sheath to facilitate the exit of filter 310 from the sheath 700 in the same manner as filter 10 described above as frictional contact between the filter 310 and the inner surface of the sheath would otherwise occur if the filter was maintained in a rigid, i.e. austenitic, condition. Once ejected from the delivery sheath or catheter 700, the filter is no longer cooled and is exposed to the warmer body temperature, which causes the filter 310 to return towards its austenitic memorized configuration.

In alternate embodiments of the foregoing filters, the strut width can vary. For example, the struts can be wider at the flared region than at the filtering portion. This is preferably achieved by removing material to create the thinner portions. These thinner portions increase the flexibility of the filter for forming the angled and curved portions upon deployment. Alternatively, the filter can have struts which are thinner, rather than wider, at the flared region than at the angled and curved regions of the filtering portion. This would provide more stability at the curved regions. The adjustment of the widths is designed to strike a balance between stability and flexibility of the various regions of the filter. Thus, other width variations are contemplated such as making multiple width changes within each strut and/or in different struts.

The filter 310 (and other filters described herein) can be inserted through the jugular vein in the neck of the patient or through the femoral vein in the leg of the patient or the arm. The filters can also be placed in the superior vena cava. It can also be removed from access through the inferior vena cava or through the internal jugular vein.

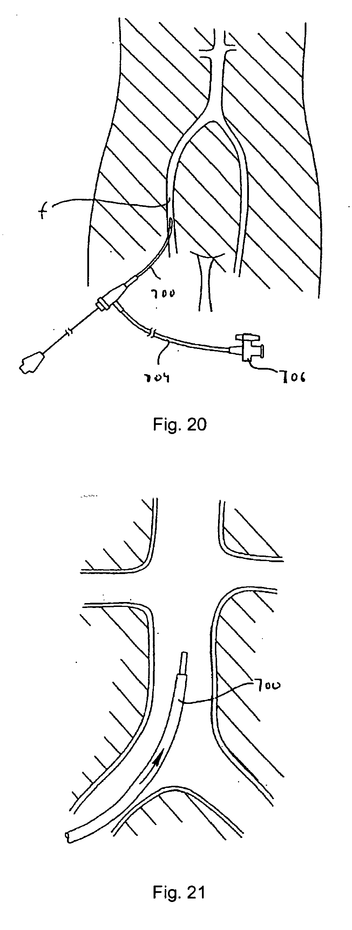

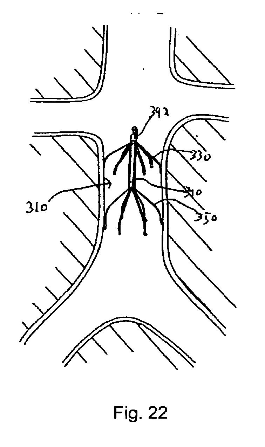

Figs. 20-31 illustrate delivery and placement of the filter 310, by way of example, in the inferior vena cava. Delivery catheter 700 is inserted through the femoral vein "f" and advanced through the iliac arteries into the inferior vena cava. Delivery catheter would be withdrawn once the tip of the sheath is adjacent the structure so that withdrawal of the sheath would place the filter in the desired location of Figure 22. Tubing 404 and valve assembly 406 enable saline injection. Delivery catheter 700 is withdrawn to enable filter 310 to be warmed by body temperature to transition to the expanded placement configuration. The other filters described herein could be inserted in the same manner. This enables blood clots or other particles to be directed to the center of the filter section by the angled struts. Thus the direction of insertion, e.g. upstream or downstream direction, will determine how the filter is to be positioned in the delivery catheter.

To facilitate removal of the foregoing filters from the vessel, cold saline can be injected onto the implanted filter or within the retrieval sheath to change the temperature of the filter to move it to a relatively softer condition to facilitate the filter being drawn into the retrieval sheath. That is, injection of cold saline will cause the filter to approach its martensitic state, bringing the filter to a more flexible condition. The flexible condition facilitates the collapse and withdrawal of the filter into the retrieval sheath by decreasing the frictional contact between the filter and the inner surface of the retrieval sheath.

While the above description contains many specifics, those specifics should not be construed as limitations on the scope of the disclosure, but merely as exemplifications of preferred embodiments thereof. For example, the filters can be inserted in other regions of the body. Also, any of the aforedescribed filters can have mounting sections of varying thickness. The foregoing filters can be made of materials other than shape memory material. Those skilled in the art will envision many other possible variations that are within the scope of the claims appended hereto.

## Claims

1. A vessel filter comprising a first region (317, 412) and a second region (315), the filter movable between a collapsed position for delivery to the vessel and an expanded position for placement within the vessel, the first region having a first set of struts (330, 414, 514) forming a first mounting portion (421, 521) and a filter portion (332, 419, 519) opening in a first direction, the filter portion having a first converging region (334, 432, 522) at a first portion to direct particles toward the center of the filter, the mounting portion being flared in the expanded position to have a transverse dimension increasing toward a second portion opposite the first portion, the second region (315) having a second set of struts (350, 454, 534, 534') forming a second mounting portion which is flared in the expanded position and opening in the first direction, **characterised in that** a plurality of spaced apart struts (340, 430) extend between the first and second regions, the struts being substantially parallel to a longitudinal axis of the filter and being formed from cutouts in the filter, the struts in the second region extending from the cutouts.

2. A vessel filter as claimed in claim 1, wherein the filter is formed from a laser cut tube (311) and composed of shape memory material.

3. A vessel filter as claimed in claim 1 or 2, wherein struts of the first set of struts (330, 414, 514) terminate in a vessel engaging hook (360a, 360b, 440a, 440b, 529a, 529b).

4. A vessel filter as claimed in any preceding claim, wherein struts of the second set of struts (534') terminate in a vessel engaging hook (570a, 570b).

5. A vessel filter as claimed in claim 3 or 4, wherein each of the vessel engaging hooks lies in a common plane with the respective strut.

6. A vessel filter as claimed in any preceding claim, wherein at least two adjacent struts of the first set of struts (414, 514) are interconnected by strut portions (414a, 414b, 514c, 514d) extending towards one another.

7. A vessel filter as claimed in claim 6, wherein the at least two adjacent struts (514a, 514b) interconnect at a first joining region (523) and then bifurcate into two interconnecting struts (514c, 514d), wherein interconnecting struts of adjacent struts join at a second joining region (524).

8. A vessel filter as claimed in any preceding claim, wherein the second region (315) further includes a retrieval region, the retrieval region including a hook (390, 415) having a cutout (393) exposing an internal annular surface (394), the annular surface dimensioned to receive a portion of a retrieval sheath.

9. A vessel filter as claimed in claim 8, wherein the retrieval region includes a radiused region (395) having first and second curved surfaces (399a, 399c) extending distally inwardly.

## Patentansprüche

1. Gefäßfilter mit einer ersten Region (317, 412) und einer zweiten Region (315), wobei das Filter zwischen einer zusammengeklappten Position zum Zuführen zu dem Gefäß und einer expandierten Position zum Platzieren in dem Gefäß beweglich ist, wobei die erste Region einen ersten Satz Streben (330, 414, 514), die einen ersten Montageteil (421, 521) bilden, und einen in einer ersten Richtung öffnenden Filterteil (332, 419, 519) aufweist, wobei der Filterteil eine erste konvergierende Region (334, 432, 522) in einem ersten Teil aufweist, um Partikel zur Mitte des Filters zu leiten, wobei der Montageteil in der expandierten Position so aufgeweitet ist, dass er eine Querabmessung hat, die in Richtung auf einen zweiten Teil gegenüber dem ersten Teil zunimmt, wobei die zweite Region (315) einen zweiten Satz Streben (350, 454, 534, 534') aufweist, die einen zweiten Montageteil bilden, der in der expandierten Position aufgeweitet ist und in der ersten Richtung öffnet, **dadurch gekennzeichnet, dass** mehrere beabstandete Streben (340, 430) zwischen der ersten und der zweiten Region verlaufen, wobei die Streben im Wesentlichen parallel zu einer Längsachse des Filters sind und von Ausschnitten im Filter gebildet werden, wobei sich die Streben in der zweiten Region von den Ausschnitten erstrecken.

2. Gefäßfilter nach Anspruch 1, wobei das Filter von einer lasergeschnittenen Röhre (311) gebildet ist und aus Formgedächtnismaterial besteht.

3. Gefäßfilter nach Anspruch 1 oder 2, wobei Streben des ersten Strebensatzes (330, 414, 514) in einem Gefäßeingriffshaken (360a, 360b, 440a, 440b, 529a, 529b) enden.

4. Gefäßfilter nach einem vorherigen Anspruch, wobei Streben des zweiten Strebensatzes (534') in einem Gefäßeingriffshaken (570a, 570b) enden.

5. Gefäßfilter nach Anspruch 3 oder 4, wobei jeder der Gefäßeingriffshaken in einer gemeinsamen Ebene mit der jeweiligen Strebe liegt.

6. Gefäßfilter nach einem vorherigen Anspruch, wobei wenigstens zwei benachbarte Streben des ersten Strebensatzes (414, 514) durch zueinander hin verlaufende Strebenteile (414a, 414b, 514c, 514d) miteinander verbunden sind.

7. Gefäßfilter nach Anspruch 6, wobei die wenigstens zwei benachbarten Streben (514a, 514b) in einer ersten Fügeregion (523) miteinander verbunden sind und sich dann in zwei miteinander verbundene Streben (514c, 514d) aufgabeln, wobei sich Verbindungsstreben von benachbarten Streben in einer zweiten Fügeregion (25) vereinigen.

8. Gefäßfilter nach einem vorherigen Anspruch, wobei die zweite Region (315) ferner eine Rückholregion beinhaltet, wobei die Rückholregion einen Haken (390, 415) mit einem Ausschnitt (393) aufweist, der eine interne ringförmige Fläche (394) exponiert, wobei die ringförmige Fläche so dimensioniert ist, dass sie einen Teil einer Rückholhülle aufnimmt.

9. Gefäßfilter nach Anspruch 8, wobei die Rückholregion eine abgerundete Region (395) mit einer ersten und einer zweiten gekrümmten Fläche (399a, 399c) aufweist, die distal einwärts verläuft.

## Revendications

1. Filtre de veine comprenant une première région (317, 412) et une deuxième région (315), le filtre étant amovible entre une position repliée pour l'administration à la veine et une position déployée pour placement à l'intérieur de la veine, la première région étant dotée d'un premier ensemble d'étais (330, 414, 514) formant une première portion de montage (421, 521) et une portion de filtre (332, 419, 519) s'ouvrant dans une première direction, la portion de filtre étant dotée d'une première région convergente (334, 432, 522) au niveau d'une première portion pour diriger des particules vers le centre du filtre, la portion de montage étant élargie dans la position déployée pour avoir une première dimension qui augmente vers une deuxième portion à l'opposé de la première portion, la deuxième région (315) étant dotée d'un deuxième ensemble d'étais (350, 454, 534, 534') formant une deuxième portion de montage qui est élargie dans la position déployée et s'ouvrant dans la première direction, **caractérisé en ce qu'**une pluralité d'étais espacés les uns des autres (340, 430) s'étendent entre les première et deuxième régions, les étais étant essentiellement parallèles à un axe longitudinal du filtre et étant formés à partir de découpes dans le filtre, les étais dans la deuxième région s'étendant depuis les découpes.

2. Filtre de veine tel que revendiqué à la revendication 1, dans lequel le filtre est formé à partir d'un tube découpé au laser (311) et composé d'un matériau à mémoire de forme.

3. Filtre de veine tel que revendiqué à la revendication 1 ou à la revendication 2, dans lequel les étais du premier ensemble d'étais (330, 414, 514) se terminent en un crochet d'engagement de veine (360a, 360b, 440a, 440b, 529a, 529b).

4. Filtre de veine tel que revendiqué dans l'une quelconque des revendications précédentes, dans lequel les étais du deuxième ensemble d'étais (534') se terminent en un crochet d'engagement de veine (570a, 570b).

5. Filtre de veine tel que revendiqué à la revendication 3 ou à la revendication 4, dans lequel chacun des crochets d'engagement de veine repose dans un plan commun avec l'étai respectif.

6. Filtre de veine tel que revendiqué dans l'une quelconque des revendications précédentes, dans lequel au moins deux étais adjacents du premier ensemble d'étais (414, 514) sont interconnectés par des portions d'étais (414a, 414b, 514c, 514d) s'étendant les unes vers les autres.

7. Filtre de veine tel que revendiqué à la revendication 6, dans lequel les au moins deux étais adjacents (514a, 514b) sont interconnectés au niveau d'une première région de jonction (523), puis bifurquent en deux étais s'interconnectant (514c, 514d), dans lequel les étais s'interconnectant d'étais adjacents se rejoignent au niveau d'une deuxième région de jonction (524).

8. Filtre de veine tel que revendiqué dans l'une quelconque des revendications précédentes, dans lequel la deuxième région (315) inclut en outre une région d'extraction, la région d'extraction incluant un crochet (390, 415) étant doté d'une découpe (393) exposant une surface annulaire interne (394), la surface annulaire étant dimensionnée pour recevoir une portion de gaine d'extraction.

9. Filtre de veine tel que revendiqué à la revendication 8, dans lequel la région d'extraction inclut une région arrondie (395) étant dotée de première et de deuxième surfaces incurvées (399a, 399c) s'étendant vers l'intérieur de façon distale.
